# EUROPÄISCHE PATENTANMELDUNG

(11) **EP 2 244 201 A1**
(43) Veröffentlichungstag der Anmeldung: **27.10.2010**
(21) Anmeldenummer: 10157671.8
(22) Anmeldetag: 25.03.2010
(51) Int. Cl.: G06F 19/00, G06F 17/30

(54) **Verfahren zur Ausgabe von medizinischen Dokumenten**

(30) Priorität: 22.04.2009 DE 102009018423
(71) Anmelder: Siemens AG Österreich, 1210 Wien (AT)
(72) Erfinder: Starke, Stefan, 1090, Wien (AT); Bauer, Gottfried, 1060, Wien (AT); Faustmann, Friedrich, 2700, Wr Neustadt (AT)
(74) Vertreter: Maier, Daniel Oliver

(57) **Zusammenfassung**

Die Erfindung betrifft ein Verfahren zur Ausgabe von medizinischen Dokumenten auf einem beliebigen Endgerät (E1, E2, E3, E4) wie z.B. PDA, Smartphone, PC, Flachbildschirm, etc. Die medizinischen Dokumente werden in einer Systemstruktur (XDS) zum Austausch medizinischer Daten generiert und archiviert, die den "Integrating the Healthcare Enterprise"-Richtlinien konform ist. Die medizinischen Dokumente sind dabei in speziellen medizin-spezifischen Datenformaten (z.B. DICOM, CDA) mit Nutz- und Metainformationen abgelegt. Nach Auswahl eines Endgeräts (E1, E2, E3, E4) wird von einem über ein Kommunikationsnetz mit diesem Endgerät (E1, E2, E3, E4) verbunden Server (DCS) ein webbasierte Dienst (WA) aufgerufen (1). Über das gewählte Endgerät (E1, E2, E3, E4) wird dann eine Patientenidentifikation eingegeben und an den Server gesendet (2), wobei vom Server ein Endgeräte-Typ festgestellt wird. Dann werden vom Server (DCS) anhand der Patientenidentifikation verfügbare, patientenspezifische medizinische Dokumente in der Systemstruktur (XDS) zum Austausch medizinischer Daten ermittelt (3-6) und die medizin-spezifischen Datenformate der jeweils ermittelten medizinischen Dokumenten vom Server (DCS) derart aufbereitet (7), dass die Nutz- und Metainformationen der medizinischen Dokumente für eine Ausgabe und Handhabung mit Hilfe des webbasierten Dienstes (WA) auf dem gewählte Endgerät (E1, E2, E3, E4) geeignet sind. Danach werden diese Dokumente dann vom Server (DCS) an das gewählte Endgerät (E1, E2, E3, E4) weitergeleitet (8, 9). Durch das erfindungsgemäße Verfahren wird auf vorteilhafte Weise und ohne zusätzlichen Aufwand eine jederzeit verfügbare Ausgabe von medizinischen Dokumenten unabhängig vom gewählten Endgerät (E1, E2, E3, E4) und voll skalierbar ermöglicht.

## Beschreibung

### Technisches Gebiet

Die vorliegende Erfindung betrifft ein Verfahren zur Ausgabe von medizinischen Dokumenten, insbesondere von Aufnahmen und Befunde aus medizinischen Fachgebieten wie z.B. Radiologie, Kardiologie und/oder Labordiagnostik, auf einem beliebigen Endgeräte - beispielsweise einem sogenannten Smartphone, einem Personal Digital Assistant (PDA), Personal Computer, Flachbildschirm, etc. Die medizinischen Dokumente werden dabei in einer "Integrating the Healthcare Enterprise" - kurz IHE - konformen Systemstruktur zum Austausch medizinischer Daten generiert und sind in dieser Systemstruktur digital in medizin-spezifischen Datenformaten abgelegt, welche Nutz- und Metainformationen umfassen.

### Stand der Technik

Heutzutage werden im Gesundheitsbereich Patientendaten (z.B. Name, Geschlecht, Alter, Sozialversicherungsnummer, etc.) sowie patientenspezifische, medizinische Dokumente (z.B. Befunde, Laborberichte, Aufnahmen und/oder Operationsberichte aus verschiedenen medizinischen Fachbereichen wie beispielsweise Radiologie, Kardiologie, Labordiagnostik, etc.) nicht mehr nur in Papierform oder auf Film archiviert, sondern sehr häufig auch in Form von digitalen Daten gespeichert. Zum Management dieser patientenspezifischen und medizinischen Informationen in digitaler Form werden daher in/von diversen Einrichtungen des Gesundheitswesens wie z.B. Krankenhäusern, Labors, Radiologie-Labors, Arztpraxen, etc. spezielle Informationstechnologie-(IT-)Systeme eingesetzt, welche auf die jeweiligen Bedürfnisse der jeweiligen Einrichtung zugeschnitten sind.

Solche IT-Systeme im Bereich des Gesundheitswesens sind beispielsweise Krankenhausinformationssysteme (KIS), Systeme für spezielle Krankenhausbereiche (z.B. Intensivstations-Informationssystem), Laborinformationsmanagementsysteme (LIMS), Radiologieinformationssysteme (RIS), Bildarchivierungs- und Kommunikationssysteme, Verwaltungssysteme für Arztpraxen (PVS), etc. Dabei umfassen beispielsweise Krankenhausinformationssysteme alle Software- und Hardwarekomponenten für eine administrative Verwaltung und für ein finanzielles Management eines Krankenhauses. In ihnen sind beispielsweise Patientendaten, Krankenakten, Behandlungspfade, Pflege- und Ressourcenplanung sowie Finanz- und Verrechnungsdaten, etc. hinterlegt. Mit Laborinformationsmanagementsystemen wird z.B. die Datenverarbeitung in chemischen, physikalischen, biologischen oder medizinischen Labors innerhalb und außerhalb eines Krankenhauses durchgeführt.

Im Bereich der Radiologie werden z.B. für Dokumentation und Verwaltung von medizinischen und administrativen Daten Radiologieinformationssysteme eingesetzt. Diese Systeme umfassen beispielsweise Funktionen wie Verwaltung von Patientendaten, Terminplanung, Dokumentation von medizinischen Daten, Bearbeitung von Abrechnungsdaten, Erstellung von radiologischen Befunden, etc. Von Radiologieinformationssystemen werden aber auch Schnittstellen zu digitalen, bildgebenden medizintechnischen Systemen wie z.B. digitales Röntgen, Computertomographen (CT), Magnetresonanztomographen (MRT), etc. bereitgestellt.

Für eine Verarbeitung und Archivierung von Bilddaten, welche von bildgebenenden medizintechnischen Systemen und Verfahren wie z.B. Röntgenanlagen, CT, MRT, Ultraschall, Endoskopie, etc. generiert werden, wird z.B. in einem Krankenhaus oder auch einem Radiologie-Labor oftmals ein Bildarchivierungs- und Kommunikationssystem mit einem gemeinsamen Speicherbereich eingesetzt. Dieses Bildarchivierungs- und Kommunikationssystem wird auch als sogenanntes Picture Archiving and Communication System (PACS) bezeichnet.

Um eine Integration verschiedener Systemkomponenten z.B. bei einem PACS-System und/oder eine Einbettung von z.B. einem PACS-System in ein Radiologie- und/oder Krankenhausinformationssystem zu ermöglichen, sind verschiedene Standards wie z.B. Health Level 7 (HL7) sowie standardisierte medizin-spezifische Datenformate und -typen (z.B. Digital Imaging and Communication in Medicine (DICOM), Clinical Document Architecture (CDA), etc.) entwickelt worden.

HL7 wird neben dem Namen für die entsprechende Organisation, von welcher diese Standards für das Gesundheitswesen entwickelt und unterstützt werden, auch als Bezeichnung für die Standards selbst verwendet. Die HL7-Standards bestehen aus einer Gruppe internationaler Standards für einen Austausch von Daten zwischen Organisationen aus dem Gesundheitsbereich (z.B. Krankenhäuser, Labors, Arztpraxen, Sozialversicherungen, etc.) und deren IT-Systemen. Vom den HL7-Standards soll eine verbesserte Interoperabilität zwischen verschiedenen Informationssystemen im Gesundheitsbereich wie z.B. Krankenhausinformationssystemen, Praxisverwaltungssystemen, Laborinformationsmanagementsystemen, Radiologieinformationssystemen, Systemen zur Leistungsverrechnung, etc. ermöglicht werden.

Im Bereich der Radiologie wird z.B. Digital Imaging and Communication in Medicine oder kurz DICOM als standardisiertes Datenformat bzw. Datentyp eingesetzt. DICOM stellt dabei einen Standard für Handhabung, Speichern, Drucken und Übertragen von digitalen Bildern in der Medizin dar, durch welchen Interoperabilität zwischen unterschiedlichen medizinischen Systemen von gegebenenfalls unterschiedlichen Herstellern ermöglicht und verbessert werden soll. DICOM wird daher z.B. von fast allen medizinischen bildgebenden Systemen und Verfahren (z.B. digitalen Röntgengeräten, CT, MRT, etc.) sowie von PACS-Systemen genutzt. Grundsätzlich gibt es bei DICOM mehrere Datenmodelle, nach denen die DICOM-Daten strukturiert sein können. Unabhängig vom jeweils verwendeten Datenmodell umfasst ein DICOM-Datensatz immer Nutzinformationen (z.B. Pixel- oder Bilddaten von digitalen Röntgengeräten, CT, MRT oder einem anderen bildgebenden Verfahren, PDF-Daten oder sonstige Binärdaten) und Metainformationen. Die Metainformation ist bei DICOM in einem sogenannten Header abgelegt und stellt Erklärungen im Bezug auf die jeweiligen Nutzinformationen bzw. Bilddaten darstellen. Solche Metainformationen umfassen beispielsweise Patientenname, Datum der Aufnahmeerstellung, Geräteparameter, Arztname, etc.

Ein von der HL7-Standardisierung erarbeitetes, standardisiertes Datenformat ist beispielsweise Clinical Document Architecture oder kurz CDA. CDA ist ein auf Extensible Markup Language (XML) basierender Standard für Austausch und Speicherung klinischer Inhalte wie z.B. Arztbriefen, Befundberichten, Medikationsplänen, etc. Ein in CDA-Datenformat gespeichertes medizinisches Dokument besteht wie ein DICOM-Dokument ebenfalls aus Nutz- und Metainformationen. Die Nutzinformationen sind z.B. in Form eines lesbaren Texts gespeicherte klinische Inhalten wie z.B. Diagnosen, Medikation, Therapien, etc. Die Metainformation können beispielsweise Patientenname, Arztname, klinische Krankheits-oder Diagnosecodes umfassen. Zusätzlich können die Nutzinformationen auch durch Informationsblöcke ergänzt sein, welche auch von Computeranwendungen verwendet werden können wie beispielsweise maschinenlesbare Angabe zur Diagnose oder Laborwerte.

Üblicherweise sind die unterschiedlichen Standards und Datenformate nur auf Teilbereiche des Gesundheitswesens zugeschnitten. DICOM wird z.B. hauptsächlich im Bereich der bildgebenden System und Verfahren eingesetzt. Daher wird von der Initiative "Integrating the Healthcare Enterprise (IHE)" versucht, den Daten- und Informationsaustausch zwischen den Rechnersystemen in Gesundheitsbereich und auf Basis der

existierenden Standards und standardisierten Datenformate (z.B. HL7, DICOM, CDA, etc.) zu harmonisieren. Bei der IHE steht eine digitale Umsetzung medizinischer Prozessabläufe zwischen den Rechnersystemen im Vordergrund. Von der IHE wurden daher Anforderungen aus der Praxis in so genannten Use Cases oder Anwendungsfälle formuliert, relevante Standards wie z.B. HL7, DICOM, CDA, etc. identifiziert und technische Leitfäden - die sogenannten Profile - für verschiedenen medizinische Fachgebiete entwickelt, mit welchen beispielsweise Produkten für das Gesundheitswesen umgesetzt und getestet werden können.

Eines dieser Profile ist eine IHE konforme Systemstruktur zum Austausch medizinischer Daten - die sogenannte Cross-Enterprise Document Sharing-Struktur (XDS). Durch diese Systemstruktur wird ein Austausch bzw. ein verteilter Zugriff auf digitale, medizinische Dokumente eines Patienten für verschiedene Institutionen von der Arztpraxis über Krankenhäuser und Notaufnahme bis zum Patientenauskunftssystem erleichtert. Die IHE konforme Cross-Enterprise Document Sharing-Struktur ist beispielsweise in den folgenden Dokumenten der IHE: "IHE IT Infrastructure Technical Framework Vol.1, Section 10, Rev. 5.0, IHE International, 2008" und "IHE IT Infrastructure Technical Framework Supplement 2007-2008, Cross-Enterprise Document Sharing XDS.b, Draft for Trial Implementation, 10.10.2008" beschrieben. Durch die Cross-Enterprise Document Sharing-Struktur nach IHE-Richtlinien wird eine Systemstruktur zur Verfügung gestellt, mit der medizinische Dokumente patientenspezifisch abgerufen werden können, wobei diese Dokumente von einem oder mehreren Informationssysteme im Gesundheitsbereich und/oder medizintechnischen bildgebenden Systemen (z.B. digitales Röntgen, CT, MRT, etc.) als Dokumentenquellen oder Document Sources generiert und zur Verfügung gestellt werden.

Die Cross-Enterprise Document Sharing-Struktur umfasst dabei eine sogenannte Document Registry, in der Informationen (z.B. Dokumentname, Typ, Referenzadresse, etc.) zum Auffinden von medizinischen Dokumenten gespeichert sind, und zumindest ein sogenanntes Document Repository. Das zumindest eine Document Repository ist für eine transparente und sichere Speicherung der medizinischen Dokumente zuständig, wobei die medizinischen Dokumente z.B. im Document Repository selbst hinterlegt sein können oder es sind Informationen gespeichert, von welchem Informations- bzw. IT-System im Gesundheitsbereich (z.B. PACS) die medizinischen Dokumenten abgerufen werden können.

Eine Abfrage der medizinischen Dokumente erfolgt bei der Cross-Enterprise Document Sharing-Struktur gemäß IHE-Richtlinien durch eine sogenannten Document Consumer. Von dem auch die gewünschten medizinischen Dokumente aus dem zumindest einen Document Repository der Cross-Enterprise Document Sharing-Struktur geholt werden. Als Document Consumer zum Abfragen und Anzeigen von medizinischen Dokumenten kann beispielsweise ein Spezialistensystem (z.B. Systeme im Krankenhausbereich, etc.), welches bereits speziell auf den Zugriff und das Anzeigen zugeschnitten ist, eingesetzt werden. Es besteht aber beispielsweise auch die Möglichkeit, dass ein Personal Computer (PC) oder ein Computersystem im Praxisbereich als Document Comsumer verwendet wird.

Ein Document Comsumer (z.B. PC, Computer in einer Arztpraxis, etc.) muss allerdings für einen Zugriff auf medizinische Dokumente der Cross-Enterprise Document Sharing-Struktur einen Zugang zum Internet ausweisen, da für eine Kommunikation mit der Document Registry und dem zumindest eine Document Repository der Cross-Enterprise Document Sharing-Struktur Standardprotokolle des Internets (z.B. Hypertext Transfer Protocol (http), etc.) und von "Electronic Business using XML" (Extensible Markup Language) eingesetzt werden. Von der Cross-Enterprise Document Sharing-Struktur abgefragte Daten bzw. die medizinischen Dokumente müssen dann vom jeweiligen Document Consumer interpretiert und für eine Anzeige aufbereitet werden.

Nachteilig wirkt sich allerdings aus, dass das als Document Consumer verwendete Endgerät (z.B. PC, etc.) für ein Anzeigen von medizinischen Dokumenten ausgestattet sind muss. Dabei können die Formate bzw. Datentypen, in welche die medizinischen Dokumente in der Cross-Enterprise Document Sharing-Struktur abgelegt sind, beispielsweise von einer einfachen Bild- oder Textdatei über medizinische Befunde im CDA-Format bis zu einer komplexen Bildfolge in DICOM reichen.

Wird beispielsweise von einem Arzt in seiner Praxis im Rahmen eines Patientengesprächs mit einem PC oder Computersystem zur Praxisverwaltung auf medizinische Dokumente der Cross-Enterprise Document Sharing-Struktur zugriffen, so müssen vorher für eine Anzeige dieser Dokumente für jedes verwendete medizin-spezifische Datenformat (z.B. CDA, DICOM) endgerätspezifische Software-Komponenten installiert werden. Es hat sich daher als nachteilig erweisen, dass für eine Funktion als Document Consumer ein Endgerät mit hohem Zusatzaufwand mit endgerätspezifischen und datenformatspezifischen Software-Komponenten ausgestattet werden muss, um medizinische Dokumente abzufragen und anzuzeigen. Zusätzlich besteht für einen Nutzer (z.B. Patient, Arzt) Wahlmöglichkeit beim Endgerät bzw. keine Möglichkeit ohne zusätzlichen Aufwand das Endgerät zu wechseln, da für einen Zugriff auf medizinische Dokumente der Cross-Enterprise Document Sharing-Struktur immer das entsprechend ausgestattete Endgerät verwendet werden muss.

### Darstellung der Erfindung

Der Erfindung liegt daher die Aufgabe zugrunde, ein Verfahren anzugeben, durch welches ohne zusätzlichen Aufwand medizinische Dokumente, welche in unterschiedliche medizin-spezifische Datenformaten gespeichert sind, auf einem beliebig gewählten Endgerät ausgegeben und an das beliebig gewählte Endgerät angepasst angezeigt werden.

Diese Aufgabe wird durch ein Verfahren der eingangs genannten Art gelöst, bei welchem zuerst ein Endgerät ausgewählt und mit dem gewählten Endgerät von einem über ein Kommunikationsnetz (z.B. Datennetz, Funknetz, etc.) mit diesem Endgerät verbunden Server ein webbasierte Dienst aufgerufen wird. Über das gewählte Endgerät wird mit Hilfe des webbasierten Dienstes eine Patientenidentifikation eingegeben und an den Server gesendet, wobei vom Server ein Endgeräte-Typ des gewählte Endgeräts (z.B. PDA, Smartphone, PC, Computer mit Flachbildschirm, etc.) festgestellt wird. Dann werden vom Server anhand der Patientenidentifikation verfügbare, patientenspezifische medizinische Dokumente in der Systemstruktur zum Austausch medizinischer Daten ermittelt und die medizin-spezifischen Datenformate der jeweils ermittelten medizinischen Dokumenten vom Server derart aufbereitet werden, dass die Nutz- und Metainformationen der medizinischen Dokumente für eine Ausgabe und Handhabung mit Hilfe des webbasierten Dienstes auf dem gewählten Endgerät geeignet sind. Danach werden diese Dokumente vom Server an das gewählte Endgerät für eine Ausgabe (z.B. in einem sogenannten Webbrowser) weitergeleitet.

Der Hauptaspekt der erfindungsgemäß vorgeschlagenen Lösung besteht darin, dass ohne zusätzlichen Aufwand - wie z.B. einer Installation einer endgerät- und/oder datenformatspezifischen Software-Komponenten von jedem beliebigen Endgeräte, insbesondere auch einem mobilen Endgeräte, medizinische Dokumente abgerufen und angezeigt werden können. Das Endgerät (z.B. PDA, Smartphone, PC, Computer mit Flachbildschirm, etc.) muss nur eine Verbindung über ein Kommunikationsnetz zum Server aufweisen, wobei vom Kommunikationsnetz einen Zugriff auf das Internet ermöglicht werden muss. Als Kommunikationsnetz können daher Datennetze mit fester Verbindung zum Server wie z.B. Local Area Networks (LANs) oder Wide Area Networks (WAN) oder Kommunikationsnetz mit einer drahtlosen Verbindung zum Server wie z.B. Funknetz (z.B. Wireless LAN, Mobilfunknetze) verwendet werden.

Durch das erfindungsgemäße Verfahren können damit medizinische Dokumente auf einfache Weise webbasiert, unabhängig vom gewählten Endgerät und voll skalierbar überall und jederzeit auf einem von einem Nutzer (z.B. Arzt, Patient) gewählten Endgerät - von einer PDA- oder Smartphone-Anzeige bis zu einem großen Flachbildschirm als Ausgabeeinheit eines zugehörigen Rechners ausgegeben werden. Es können damit auf dem Endgerät medizinische Dokumente, welche in medizin-spezifischen Datenformaten (z.B. CDA, DICOM) je nach gewähltem Endgerät ohne bzw. mit sehr geringem Informationsverlust und entsprechend dem jeweiligen Datentyp der Nutz- und Metainformation als Bild, Video, Text, etc. dargestellt werden.

Bei einer bevorzugten Fortbildung des erfinderischen Verfahrens werden vom Server anhand der eingegebenen Patientenidentifikation zuerst Referenzadressen der verfügbaren, patientenspezifischen medizinischen Dokumente in der Systemstruktur zum Austausch medizinischer Daten ermittelt. Anhand der Referenzadressen wird vom Server eine Auswahlliste der verfügbaren medizinischen Dokumente erstellt und mit Hilfe des webbasierten Dienstes auf dem gewählten Endgerät angezeigt. Nach Auswahl von zumindest einem medizinischen Dokument aus der Auswahlliste wird das gewählte Dokument vom Server anhand einer zugehörigen Referenzadresse in der Systemstruktur zum Austausch medizinischer Daten abgerufen und derart umgewandelt, dass die Nutz- und Metainformation des gewählten Dokuments mit Hilfe des webbasierten Dienstes auf dem gewählten Endgerät anzeigbar und handhabbar sind. Damit wird auf einfache Weise eine Suche nach gewünschten medizinischen Endgeräten verkürzt und für das gewählte Endgerät übersichtlicher gestaltet. Eine Ausgabe auf dem gewählten Endgerät wird damit zusätzlich beschleunigt, da zuerst bei der Systemstruktur zum Austausch medizinischer Daten wie z.B. der Cross-Enterprise Document Sharing-Struktur nur auf die Document Registry zugegriffen werden muss. Von der Document Registry werden dem Server dann die Referenzadressen zum Erstellen der Auswahlliste geliefert, wobei die Referenzadressen Informationen wie z.B. Dokumentname, Datenformat des Dokuments, Datum, medizinisches Fachgebiet, Speicheradresse innerhalb der Systemstruktur, etc. enthalten können.

Es ist vorteilhaft, wenn die Auswahlliste der ermittelten medizinischen Dokumente auf dem gewählten Endgerät strukturiert nach Datum, nach Typ der Nutzinformation der medizinischen Dokumente und/oder nach medizinischen Kriterien, insbesondere Fachgebiet, Medikamentenverwendung, behandelnder Arzt oder behandelndes Krankenhaus, ausgegeben wird. Auf diese einfache Weise können vom Nutzer (z.B. Arzt, Patient) die für ihn interessanten patientenspezifischen Dokumente - insbesondere bei Endgeräten mit eher kleiner Ausgabeeinheit wie z.B. PDAs, Smartphones, etc. rascher für eine Auswahl gefunden werden. Von Nutzer brauchen beispielsweise nur mehr für ihn interessante Zeiträume, Typen der Nutzinformation (z.B. Bilder, Video, Text) oder medizinische Fachgebiete betrachtet werden.

Es ist auch günstig, wenn die Ausgabe der patientenspezifischen medizinischen Dokumente durch den Server anhand von über den webbasierten Dienst eingebbaren Suchkriterien vorgefiltert wird. Eine derartige Vorfilterung ist insbesondere bei Endgeräten mit eher kleiner Ausgabeeinheit wie z.B. PDAs, Smartphones, etc. von Vorteil, da dann eine kürzere Auswahlliste auf dem Endgerät angezeigt wird und damit die Suche nach einem gewünschten medizinischen Dokument für den Nutzer vereinfacht wird. Auch bei langen und/oder umfangreichen Patientenakten kann auf diese Weise die Auswahlliste beispielsweise auf für den Nutzer interessante Zeiträume, Fachgebiete, etc. der medizinischen Dokumente bzw. auch Dokumenttypen eingeschränkt werden.

Zweckmäßigerweise wird zusätzlich zur Patientenidentifikation für ein Ausgeben, Anzeigen und Handhaben der medizinischen Dokumente auch eine Arztberechtigung durchgeführt. Damit kann vorteilhaft und in Abstimmung mit diversen nationaler Datenschutzregelungen geprüft werden, ob der jeweilige Nutzer (z.B. Arzt) berechtigt ist, ein bestimmtes medizinisches Dokument eines Patienten einzusehen. Durch eine Überprüfung einer Arztberechtigung kann auf einfache Weise verhindert werden, dass Datenschutzregeln oder die Privatsphäre eines Patienten verletzt wird. Da medizinische Dokumente wie z.B. Befunde, Krankenberichte, Diagnosen, etc. besonders sensible Daten sind, kann z.B. vom jeweiligen Patient festgelegt werden, welcher Arzt oder welches Krankenhaus, etc. berechtigt ist, bestimmte medizinische Dokumente einzusehen. Dabei ist es von Vorteil, wenn die Arztberechtigung zum Anzeigen und Handhaben der medizinischen Dokumente z.B. vom Patienten zeitlich befristet eingerichtet und bestätigt werden kann.

Auf diese Weise kann von einem Patient beispielsweise in einer Notfallsituation einem behandelnden Arzt (z.B. Notarzt) für eine bestimmte Zeitspanne Einsicht in wichtige medizinische Dokumente gewährt werden. Eine Befristung kann beispielsweise durch Eingabe einer Gültigkeitsdauer für die Berechtigung des Arztes erreicht werden. Ein Arzt kann z.B. auch zeitlich befristeten Einblick durch eines paralleles Aufrufen des webbasierten Dienstes vom Server durch Patient und Arzt und ein nachfolgendes Freischalten der Arztberechtigung des Arztes durch den Patienten für seine medizinischen Dokumente Einblick erhalten. Nach Beendigung des webbasierten Dienstes durch den Patienten kann dem Arzt die zeitlich befristete Berechtung zur Einsicht der patientenspezifischen medizinischen Dokumente wieder entzogen werden.

Es ist vorteilhaft, wenn die Patientenidentifikation und/oder die Arztberechtigung von einer Identitätskarte eingelesen werden oder als Kennwort und persönlicher Identifikationsnummer (PIN) über den webbasierten Dienst eingegeben werden. Vom webbasierten Dienst kann vom Nutzer (z.B. Patient, Arzt) z.B. eine Eingabe eines Kennworts und einer persönlichen Identifikationsnummer (PIN) über eine Tastatur des Endgeräts gefordert werden. Alternativ besteht die Möglichkeit die Patientenidentifikation und/oder die Arztberechtigung von einer Identifikationskarte (z.B. elektronischen Gesundheitskarte, etc.) bei Verwendung eines IC-Chips mittels Kartenlesers oder bei einem Barcodes mittels Barcodeleser einzulesen. Aus Sicherheitsgründen kann der Nutzer zusätzlich zur Eingabe eines PINs aufgefordert werden.

Eine zweckmäßige Weiterbildung des erfindungsgemäßen Verfahrens sieht vor, dass vom webbasierten Dienst für die endgeräte-spezifische Anzeige der medizinischen Dokumente eine sogenannte JavaServer Faces-Technologie eingesetzt wird. JavaServer Faces ist ein sogenannter Framework-Standard zur Entwicklung von Benutzeroberflächen für webbasierte Dienste. Durch den Einsatz der JavaServer Faces-Technologie können für den webbasierten Dienst des erfindungsgemäßen Verfahrens auf einfache Weise skalierbare Benutzeroberflächen für die Anzeige auf einem beliebig gewählten Endgerät entwickelt werden. Die Benutzeroberfläche kann dadurch auf eine Anzeigeeinheit des beliebig gewählten Endgeräts von z.B. einem PDA oder Smartphone über einen PC mit Bildschirm bis zu einem Computer mit einem Flachbildschirm bzw. einer Flachbildschirm-Leinwand angepasst werden.

Es ist günstig, wenn vom Server medizin-spezifische Datenformate wie Digital Imaging and Communications (DICOM) in Medicine und/oder Clinical Document Architecture (CDA) umgewandelt werden, da sowohl DICOM als auch CDA standardisierte und im Gesundheitswesen häufig verwendete Datenformate sind. DICOM ist ein standardisiertes Datenformat bzw. ein Standard für Handhabung, Speichern, Drucken und Übertragen von digitalen Bildern in der Medizin, welches insbesondere von medizinischen bildgebenden Systemen und Verfahren (z.B. digitalen Röntgengeräten, CT, MRT, Ultraschall, Endoskopie, etc.) sowie von PACS-Systemen, in welche digitale Aufnahme aus dem Bereich der Radiologie archiviert werden, genutzt wird. CDA ist ein vom der HL7-Standardisierung erarbeitetes standardisiertes Datenformat bzw. ein auf Extensible Markup Language (XML) basierender Standard für den Austausch und die Speicherung klinischer Inhalte. Im CDA-Datenformat werden beispielsweise medizinische Dokumente wie Arztbriefe, Befundberichte, klinische Diagnosen, Medikationspläne, etc. gespeichert.

### Kurzbeschreibung der Zeichnung

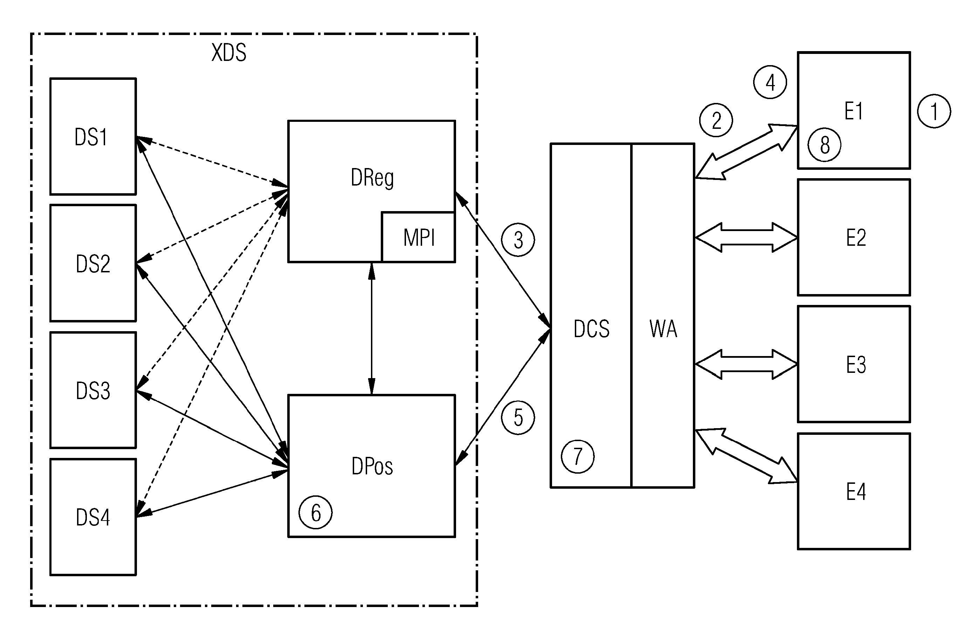

Die Erfindung wird nachfolgend in beispielhafter Weise anhand der beigefügten Figur 1 erläutert. Es zeigt Figur 1 beispielhaft den Ablauf des erfindungsgemäßen Verfahrens zur Ausgabe von medizinischen Dokumenten.

### Ausführung der Erfindung

Figur 1 zeigt in schematischer und beispielhafter Weise eine Systemstruktur XDS zum Austausch medizinischer Daten, welche konform zu Richtlinien der "Integrating the Healthcare Enterprise (IHE)"-Initiative ist. Eine derartige Systemstruktur XDS ist beispielsweise die sogenannte Cross-Enterprise Document Sharing-Struktur, welche z.B. in den Dokumenten IHE IT Infrastructure Technical Framework Vol.1, Section 10, Rev. 5.0, IHE International, 2008 und IHE IT Infrastructure Technical Framework Supplement 2007-2008, Cross-Enterprise Document Sharing XDS.b, Draft for Trial Implementation, 10.10.2008 beschrieben wird.

Die Systemstruktur XDS zum Austausch medizinischer Daten umfasst zumindest ein Dokumentenregister DReg, zumindest ein zentrales, elektronisches Dokumentenarchiv DPos sowie mehrere Dokumentquellen DS1, DS2, DS3, DS4, von welchen die medizinischen Dokumente generiert und zur Verfügung gestellt werden.

Im Dokumentenregister DReg, welches bei einer Cross-Enterprise Document Sharing-Struktur auch als Document Registry bezeichnet wird, sind z.B. Referenzadressen der medizinischen Dokumente hinterlegt. Diese Referenzadressen behalten beispielsweise Basisinformationen zu einem medizinischen Dokument wie z.B. Dokumentname, Datenformat des Dokuments, Datum, medizinisches Fachgebiet, Speicheradresse innerhalb der Systemstruktur XDS, etc. Zusätzlich kann das Dokumentenregister DReg auch ein Hauptverzeichnis MPI von Patientenidentifikationen aufweisen, welches Inhalte zur eindeutigen Identifikation eines Patienten wie beispielsweise Patientennamen, eine eindeutige Patientennummer (z.B. Sozialversicherungsnummer), etc. umfasst. Das Hauptverzeichnis MPI der Patientenidentifikationen kann z.B. auch als Master Patient Index MPI bezeichnet werden.

Im zumindest einen zentralen Dokumentenarchiv DPos, welches bei einer Cross-Enterprise Document Sharing-Struktur auch als Document Repository bezeichnet wird, sind die von den Dokumentquellen DS1, DS2, DS3, DS4 zur Verfügung gestellten medizinischen Dokumente hinterlegt. Zusätzlich ist das Dokumentenarchiv DPos auch für eine Registrierung neuer medizinischer Dokumente im Dokumentenregister DReg zuständig. Dokumentquellen DS1, DS2, DS3, DS4, von welchen medizinische Dokumente generiert und/oder zur Verfügung gestellt werden, sind beispielsweise Krankhaus- oder Laborinformationssysteme, bildgebende medizinische Systeme (z.B. digitale Röntgenanlagen, CT, MRT, etc.) und/oder Bildarchivierungs- und Kommunikationssysteme (z.B. PACS).

Von diesen Dokumentquellen DS1, DS2, DS3, DS4 werden medizinische Dokumente wie z.B. Röntgen-, CT-, MRT-Aufnahmen, etc. sowie Arztbriefe, Befundberichte, klinische Diagnosen, Medikationspläne, etc. generiert. Die medizinischen Dokumente werden dann dem zumindest einen zentralen Dokumentenarchiv DPos der Systemstruktur XDS zum Austausch von medizinischen Daten zur Verfügung gestellt. D.h. die medizinischen Dokumente werden entweder direkt im zentralen Dokumentenarchiv DPos gespeichert oder es wird, insbesondere bei medizinischen Dokumenten, welche große Datenmengen umfassen (z.B. z.B. Röntgen-, CT-, MRT-Aufnahmen, etc.), ein Speicherinformation zum Auffinden des jeweiligen medizinischen Dokuments im zentralen Dokumentenarchiv DPos hinterlegt. Für jedes zur Verfügung gestellte medizinische Dokument wird vom zentralen Dokumentenarchiv DPos eine eindeutige Referenzadresse generiert, welche an das Dokumentenregister DReg weitergeleitet und dort gespeichert wird.

Dokumentenregister DReg, das zumindest eine zentrale Dokumentenarchiv DPos sowie die Dokumentenquellen DS1, DS2, DS3, DS4 der Systemstruktur XDS sind für Kommunikation und Datenaustausch über ein Netz (z.B. Internet) miteinander verbunden und befinden sich üblicherweise bei z.B. verschiedenen Krankenhäusern, Labors, Arztpraxen und/oder weiteren Einrichtung des Gesundheitswesens. Von der IHE konformen Systemstruktur XDS zum Austausch von medizinischen Daten werden medizinische Dokumente beispielsweise neutral behandelt. Das heißt, die medizinischen Dokumente werden unabhängig vom jeweiligen medizin-spezifischen Datenformat registriert, abgelegt und verwaltet. So werden von einer Cross-Enterprise Document Sharing-Struktur beispielsweise gängige medizin-spezifische Datenformate wie DICOM, CDA, etc. unterstützt, welche neben Nutzinformationen (z.B. Röntgenbild, CT- oder MRT-Aufnahmen, Befund in Textform) auch Metainformationen (z.B. Zusatzdaten zu Röntgen-, CT-, MRT-Bildern, kodierte klinische Informationen, etc.) umfassen.

Mit der Systemstruktur XDS zum Austausch medizinischer Daten ist ein Server DCS z.B. über ein Kommunikationsnetz (z.B. Internet) verbunden. Vom Server DCS wird über diese Kommunikationsnetz beispielweise über Internetprotokolle wie z.B. HTTP, HTTPs oder XML mit dem Dokumentenregister DReg und dem zumindest eine zentralen Dokumentenarchiv DPos kommuniziert.

Auf den Server, welcher ein webbasierter Dienst WA für Zugriff auf und Ausgabe von medizinischen Dokumenten umfasst, kann über ein Kommunikationsnetz mit Hilfe von Endgeräten E1, E2, E3, E4 zugegriffen und damit der webbasierte Dienst aufgerufen werden. Als Endgeräte E1, E2, E3, E4 für einem Nutzer mobile Endgeräte können wie z.B. PDA, Smartphone, Laptop oder PC mit Bildschirm oder Flachbildschirm als Ausgabeeinheit vorgesehen sein. Die Endgeräte E1, E2, E3, E4 weisen eine Verbindung über ein drahtgebundenes (z.B. LAN, WAN) oder drahtloses (z.B. Wireless LAN, Mobilfunknetz) zum Server DCS auf, wobei von diesem Kommunikationsnetz ein Zugang zum Internet ermöglicht werden muss. Die Kombination aus dem Server DCS mit dem webbasierten Dienst WA und einem jeweils von einem Nutzer beliebig gewählten Endgerät E1, E2, E3, E4 wird im Sinne der IHE Cross-Enterprise Document Sharing-Struktur auch als Imaging Document Consumer bezeichnet.

Für eine Ausgabe von medizinischen Dokumenten, die in der Systemstruktur XDS zum Austausch von medizinischen Dokumenten archiviert sind, wird in einem ersten Verfahrensschritt 1 von einem Nutzer (z.B. Patient, Arzt) ein beliebiges, Internetfähiges Endgerät E1, E2, E3, E4 gewählt, welches dem Nutzer gerade zur Verfügung steht. Durch Anwahl des Servers DCS über das Internet wird der am Server DCS gespeicherte webbasierte Dienst WA aufgerufen und beispielsweise auf einer Ausgabeeinheit (z.B. Anzeigeeinheit von PDA oder Smartphone, PC-Bildschirm, Flachbildschirm) des gewählten Endgeräts E1, E2, E3, E4 eine Startseite das webbasierten Dienstes WA in einem sogenannten Webbrowser angezeigt.

In einem zweiten Verfahrensschritt 2 wird dann vom Nutzer die Eingabe einer Identifikation (z.B. Patientenidentifikation, Arztberechtigung) gefordert. Die Patientenidentifikation bzw. Arztberechtigung kann z.B. über eine mit dem gewählten Endgerät E1, E2, E3, E4 verbundene Tastatur in Form eines Kennworts (z.B. Sozialversicherungsnummer, Patientenname, Arzt-ID, Arztname, etc.) und PINs eingegeben werden. Alternativ kann im zweiten Verfahrensschritt 2 eine Eingabe der Patientenidentifikation bzw. der Arztberechtigung z.B. durch Einlesen eines Barcodes oder durch Auslesen eine IC-Chip erfolgen, wobei der Barcode bzw. der IC-Chip auf der Identitätskarte (z.B. elektronische Gesundheitskarte, elektronische Krankenversicherungskarte, etc.) angebracht sein können, welche Daten wie z.B. Patientenname, Sozialversicherungsnummer, etc. umfasst. Diese Möglichkeiten stehen allerdings nur dann zur Identifikation von Patient bzw. Arzt zur Verfügung, wenn entsprechende Eingabegeräte wie z.B. Kartenleser oder Barcodeleser bei dem gewählten Endgerät E1, E2, E3, E4 verfügbar oder anschließbar sind. Aus Sicherheitsgründen kann zusätzlich nach Einlesen der Patientenidentifikation bzw. der Arztberechtigung noch eine Eingabe eines PINs gefordert werden. Zusätzlich wird vom Server DCS im zweiten Verfahrensschritt festgestellt, welche Endgerät E1, E2, E3, E4 für einen Zugriff auf die medizinischen Dokumente gewählt worden ist.

Nach Eingabe bzw. Einlesen von Kennwort und PIN wird die eingegebene Patientenidentifikation bzw. die Arztberechtigung an den Server DCS weitergeleitet und in einem dritten Verfahrensschritt 3 wird dann vom Server DCS eine Suchabfrage im Dokumentenregister DReg der Systemstruktur XDS zum Austausch medizinischer Daten gestartet. Bei einer eingegebenen Arztberechtigung werden im Dokumentenregister DReg bzw. im Master Patient Index MPI zuerst alle jene Patientenidentifikationen (z.B. Sozialversicherungsnummer) ermittelt, für welche der Arzt bzw. die Ärztin über eine Berechtigung zur Einsicht der zugehörigen medizinischen Dokumente verfügt. Dem Arzt bzw. der Ärztin werden nach Eingabe der Arztberechtigung zuerst alle jene Patientenidentifikationen - z.B. als Auswahlliste - angezeigt, für die er/sie berechtigt ist. Vom Arzt bzw. von der Ärztin kann nach Prüfung der Arztberechtigung die gewünschte Patientenidentifikation gewählt werden, welche an den Server DCS gesendet wird und welche von einem Patient als Nutzer zur Einsicht seiner/ihre medizinischen Dokument gleich im Verfahrensschritt 2 eingegeben wird. Zusätzlich besteht auch die Möglichkeit, dass ein Patient z.B. in einem Notfall eine Arzt bzw. einer Ärztin zeitlich befristet eine Berechtigung zur Einsicht in seine/ihre medizinischen Dokumente erteilt.

Im dritten Verfahrensschritt 3 werden dann im Dokumentenregister DReg anhand der eingelesenen oder vom Arzt/von der

Ärztin gewählten Patientenidentifikation die verfügbaren, medizinischen Dokumente des Patienten bzw. die entsprechenden Referenzadressen dieser Dokumente ermittelt. In einem vierten Verfahrensschritt 4 werden dann vom Dokumentenregister DReg die zur Patientenidentifikation gefundenen Referenzadressen der medizinischen Dokumente an den Server DCS zurückgesendet. Vom Server DCS werden die ermittelten Referenzadressen der medizinischen Dokumente an das gewählte Endgerät E1, E2, E3, E4 weitergeleitet und dort mit Hilfe des webbasierten Dienstes WA als Auswahlliste der für einen Patienten verfügbaren, medizinischen Dokumente auf der Ausgabeeinheit (z.B. Anzeige, Bildschirm, etc.) des gewählten Endgeräts E1, E2, E3, E4 z.B. in einem Webbrowser und angepasst an die Größe der Ausgabeeinheit des gewählten Endgeräts E1, E2, E3, E4 angezeigt. Die Referenzadresse eines medizinischen Dokuments umfasst dazu neben einer Speicheradresse des Dokuments innerhalb der Systemstruktur XDS auch Informationen wie z.B. Dokumentname, Datenformat des Dokuments (z.B. Text, Bild, Video, etc.), Datum, medizinisches Fachgebiet, etc. Zusätzlich kann die auf dem gewählten Endgerät E1, E2, E3, E4 angezeigte Auswahlliste der ermittelten medizinischen Dokumente z.B. nach Datum, Typ einer enthaltenen Nutzinformation (z.B. Text, Bild, Video, etc.) oder nach medizinischen Kriterien (z.B. Fachgebiet, Medikamentenverwendung, behandelnder Arzt, behandelndes Krankenhaus, etc.) strukturiert sein, um gewünschte medizinische Dokumenten leichter aufzufinden.

Nach Auswahl eines gewünschten medizinischen Dokuments aus der am gewählten Endgerät E1, E2, E3, E4 angezeigten Auswahlliste wird in einem fünften Verfahrensschritt 5 das ausgewählte medizinische Dokument dem Server DCS mitgeteilt. Vom Server DCS wird dann die bereits im dritten Verfahrensschritt 3 ermittelte, zugehörige Referenzadresse ausgewertet und einen entsprechende Anforderung für dieses medizinische Dokument an das zentrale Dokumentenarchiv DPos gesendet. Vom zentralen Dokumentenarchiv DPos wird in einem sechsten Verfahrensschritt 6 das gewünschte medizinische Dokumente von der jeweiligen Speicheradresse - entweder im zentralen Dokumentenarchiv DPos selbst oder bei einer der Dokumentenquellen DS1, DS2, DS3, DS4 (z.B. PACS) - geholt und an den Server DCS gesendet.

In einem siebten Verfahrensschritt 7 werden dann die medizinischen Dokumente, welche in einem medizin-spezifischen Datenformat (z.B. DICOM, CDA) in Form von Nutz- und Metainformationen abgelegt und an den Server DCS geliefert worden sind, vom Server DCS für eine Ausgabe mit Hilfe des webbasierten Dienstes WA auf dem gewählten Endgerät E1, E2, E3, E4 aufbereitet. Das bedeutet, die medizinischen Datenformate werden derart konvertiert, dass die im jeweiligen Dokument enthaltenen Nutzinformationen und Metainformationen in einem achten Verfahrensschritt 8 auf dem gewählten Endgerät E1, E2, E3, E4 durch den webbasierten Dienst WA z.B. in einem endgeräte-spezifischen Webbrowser (z.B für Nutzinformationen in Bildform) oder mittels am Endgerät verfügbarer Software-Anwendungen (z.B. Windows Media Player, Quicktime, etc. für Video; Adobe Acrobat Reader, MS Office für Textdaten; etc.) angezeigt und manipuliert werden können.

Die in den medizinischen Dokumenten in medizin-spezifischen Datenformaten (z.B. CDA, DICOM) abgelegten Nutz- und Metainformationen werden im siebten Verfahrensschritt 7 vom Server DCS daher endgeräte-spezifisch in Standard-Text-, Standard-Bild- und/oder Standard-Videoformate wie beispielsweise pdf, jpeg, Bitmap, mpeg, Audio Video Interleave (AVI), etc. umgewandelt. Metainformationen werden dabei z.B. als Text, ergänzende Information, etc. sichtbar gemacht. Bei medizinischen Dokumenten im CDA-Format (z.B. Arztbriefen, Befunden, etc.), welche weitere medizinische Dokumente als sogenannte eingebettete Dateien enthalten können, werden beispielsweise diese eingebetteten Dateien ausgewertet. D.h. vom Server DCS werden die entsprechenden Inhalte der eingebetteten Dateien in der Systemstruktur XDS zum Austausch medizinischer Daten ermittelt und ebenfalls für die Ausgabe auf dem gewählten Endgerät E1, E2, E3, E4 aufbereitet - sofern dies aufgrund der Größe der Anzeige der Ausgabeeinheit des gewählten Endgeräts E1, E2, E3, E4 sinnvoll ist.

Im achten Verfahrensschritt 8 werden dann die vom Server DCS entsprechend aufbereiteten medizinischen Dokumente an das gewählte Endgerät E1, E2, E3, E4 weitergeleitet und ein Inhalt der medizinischen Dokumente auf der jeweiligen Ausgabeeinheit des gewählten Endgeräts E1, E2, E3, E4 mit Hilfe des webbasierten Dienstes WA ausgegeben. D.h. die medizinischen Dokumenten werden entweder mit Hilfe des webbasierten Dienstes WA in einem endgeräte-spezifischen Webbrowser angezeigt oder es wird mit Hilfe des webbasierten Dienstes auf dem gewählten Endgerät E1, E2, E3, E4 eine verfügbare und entsprechende Software-Anwendungen (z.B. Windows Media Player, Quicktime, etc. für Video; Adobe Acrobat Reader, MS Office für Textdaten; etc.) für eine Ausgabe des medizinischen Dokuments gestartet.

Zusätzlich ist beim erfindungsgemäßen Verfahren für medizinische Dokumente im DICOM-Format zum Anzeigen von Nutzinformationen (z.B. Bilder, Video, etc.) und in einem sogenannten Header abgelegten Metainformationen (z.B. Patientenname, Datum der Aufnahmeerstellung, Geräteparameter, Arztname, etc.) eine speziell auf das DIOM-Format zugeschnittene Anwendung (= ein sogenannter DICOM-Viewer) vorgesehen, falls ein PC oder Computer mit Flachbildschirm als Endgerät E1, E2, E3, E4 für die Ausgabe von medizinischen Dokumenten gewählt worden ist. Der DICOM-Viewer ist eine als sogenanntes Java-Applet gestaltete Anwendung, die in den webbasierten Dienst WA voll integriert ist. Ein Java-Applet ist eine Anwendung, welche in der Programmiersprache Java verfasst worden ist und üblicherweise in einem Webbrowser ausgeführt werden kann. Wird vom Server DCS im zweiten Verfahrensschritt 2 als gewähltes Endgerät E1, E2, E3, E4 ein PC oder Computer mit Flachbildschirm - also ein Endgerät E1, E2, E3, E4 mit einer relativ großflächigen Ausgabeeinheit - und im siebten Verfahrensschritt 7 DICOM als Datenformat des gewählten medizinischen Dokuments festgestellt, so wird für die Ausgabe der medizinischen Dokumente im achten Verfahrensschritt 8 der DICOM-Viewer an das gewählte Endgerät E1, E2, E3, E4 für die Ausgabe mitgesendet. Vom DICOM-Viewer wird auf vorteilhafte Weise auch das Datenmanagement für die an das Endgerät E1, E2, E3, E4 zu übertragenden Nutz- und Metainformationen des medizinischen Dokumentes im DICOM-Format übernommen.

Durch das erfindungsgemäße Verfahren können damit überall und jederzeit von einem beliebigen bzw. gerade verfügbaren Endgerät E1, E2, E3, E4 medizinische Dokumente z.B. bei Patientenrückfragen, Besprechungen, etc. auf einfache Weise und ohne Zusatz eingesehen werden.

## Patentansprüche

1. Verfahren zur Ausgabe von medizinischen Dokumenten auf einem Endgerät (E1, E2, E3, E4), wobei die medizinischen Dokumente in einer "Integrating the Healthcare Enterprise" konformen Systemstruktur (XDS) zum Austausch medizinischer Daten generiert und archiviert werden und in medizin-spezifischen Datenformaten mit Nutz- und Metainformationen abgelegt sind, **dadurch gekennzeichnet, dass** zuerst ein Endgerät (E1, E2, E3, E4) ausgewählt und mit dem gewählten Endgerät (E1, E2, E3, E4) von einem über ein Kommunikationsnetz mit diesem Endgerät verbundenen Server (DCS) ein webbasierte Dienst (WA) aufgerufen wird (1),
dass dann über das gewählte Endgerät (E1, E2, E3, E4) eine Patientenidentifikation eingegeben und an den Server (DCS) gesendet wird, wobei vom Server (DCS) ein Endgeräte-Typ des gewählte Endgerät (E1, E2, E3, E4) festgestellt wird (2),
dass dann vom Server (DCS) anhand der Patientenidentifikation verfügbare, patientenspezifische medizinische Dokumente in der Systemstruktur (XDS) zum Austausch medizinischer Daten ermittelt werden (3-6), und dass dann die medizin-spezifischen Datenformate der jeweils ermittelten medizinischen Dokumenten vom Server (DCS) derart aufbereitet werden (7), dass die Nutz- und Metainformationen der medizinischen Dokumente für eine Ausgabe und Handhabung mit Hilfe des webbasierten Dienstes (WA) auf dem gewählte Endgerät (E1, E2, E3, E4) geeignet sind, und dass diese Dokumente dann vom Server (DCS) an das gewählte Endgerät (E1, E2, E3, E4) weitergeleitet werden (8, 9).

2. Verfahren nach Anspruch 1, **dadurch gekennzeichnet, dass** vom Server (DCS) anhand der eingegebenen Patientenidentifikation zuerst Referenzadressen der verfügbaren, patientenspezifischen medizinischen Dokumente in der Systemstruktur (XDS) zum Austausch medizinischer Daten ermittelt werden (3), dass dann vom Server (DCS) anhand der Referenzadressen eine Auswahlliste der verfügbaren medizinischen Dokumente erstellt und mit Hilfe des webbasierten Dienstes (WA) auf dem gewählten Endgerät (E1, E2, E3, E4) angezeigt wird (4), dass nach Auswahl zumindest eines medizinischen Dokuments aus der Auswahlliste das gewählte Dokument vom Server (DCS) anhand einer zugehörigen Referenzadresse in der Systemstruktur (XDS) zum Austausch medizinischer Daten abgerufen (5, 6) und derart umgewandelt wird (7), dass die Nutz- und Metainformation des gewählten Dokuments mit Hilfe des webbasierten Dienstes (WA) auf dem gewählten Endgerät (E1, E2, E3, E4) anzeigbar und handhabbar sind.

3. Verfahren nach einem der Ansprüche 1 bis 2, **dadurch gekennzeichnet, dass** die Auswahlliste der ermittelten medizinischen Dokumente auf dem gewählten Endgerät (E1, E2, E3, E4) strukturiert nach Datum, nach Typ der Nutzinformation der medizinischen Dokumente und/oder nach medizinischen Kriterien, insbesondere Fachgebiet, Medikamentenverwendung, behandelnder Arzt oder behandelndes Krankenhaus, ausgegeben wird (8).

4. Verfahren nach einem der Ansprüche 1 bis 3, **dadurch gekennzeichnet, dass** die Ausgabe der patientenspezifischen medizinischen Dokumente durch den Server anhand von über den webbasierten Dienst (WA) eingebbaren Suchkriterien vorgefiltert wird.

5. Verfahren nach einem der Ansprüche 1 bis 4, **dadurch gekennzeichnet, dass** zusätzlich für ein Anzeigen und Handhaben der medizinischen Dokumente eine Überprüfung einer Arztberechtigung durchgeführt wird (2, 3).

6. Verfahren nach Anspruch 5, **dadurch gekennzeichnet, dass** die Arztberechtigung zum Anzeigen und Handhaben der medizinischen Dokumente zeitlich befristet eingerichtet wird.

7. Verfahren nach einem der Ansprüche 1 bis 6, **dadurch gekennzeichnet, dass** die Patientenidentifikation und/oder die Arztberechtigung von einer Identitätskarte eingelesen werden oder als Kennwort und persönlicher Identifikationsnummer über den webbasierten Dienst (WA) eingegeben werden.

8. Verfahren nach einem der Ansprüche 1 bis 7, **dadurch gekennzeichnet, dass** vom webbasierten Dienst (WA) für die endgeräte-spezifische Anzeige der medizinischen Dokumente eine sogenannte JavaServer Faces Technologie eingesetzt wird.

9. Verfahren nach einem der Ansprüche 1 bis 8, **dadurch gekennzeichnet, dass** von Server (DCS) medizin-spezifische Datenformate wie Digital Imaging and Communications in Medicine und/oder Clinical Document Architecture umgewandelt werden (7).
